# EUROPEAN PATENT APPLICATION

(11) **EP 4 792 767 A1**
(43) Date of publication of application: **19.08.2026**
(21) Application number: 24876628.9
(22) Date of filing: 11.10.2024
(51) Int. Cl.: A61B 34/20, A61B 34/30

(54) **ELECTROMAGNETIC NAVIGATION METHOD FOR SURGICAL ROBOT**

(30) Priority: 11.10.2023 CN 202311315344
(71) Applicant: CHUNFENGHUAYU (SUZHOU) INTELLIGENT MEDICAL TECHNOLOGY CO., LTD., Taicang Suzhou, Jiangsu 215413 (CN)
(72) Inventor: YOUNG, Victor, Suzhou, Jiangsu 215413 (CN); CHEN, Chaoliang, Suzhou, Jiangsu 215413 (CN); SHI, Weisong, Suzhou, Jiangsu 215413 (CN)
(74) Representative: Maiwald GmbH
(86) International application number: PCT/CN2024/124100
(87) International publication number: WO 2025/077817

(57) **Abstract**

The present invention relates to an electromagnetic navigation method for a surgical robot. The electromagnetic navigation method comprises steps of: S1, providing a reference frame device; S2, scanning the reference frame by means of an intraoperative medical imaging apparatus, so as to obtain the coordinates of a developing device in an intraoperative medical imaging apparatus space; S3, according to said coordinates and the coordinates of the developing device in an electromagnetic space, calculating a first transformation matrix for the transformation from the coordinates in the electromagnetic space to the coordinates in the intraoperative medical imaging apparatus space; S4, by means of the first transformation matrix, transforming surgical operation information in the intraoperative medical imaging apparatus into surgical operation information in the electromagnetic space; S5, acquiring the coordinates of a surgical tool in the electromagnetic space and the coordinates of the surgical tool in a robot coordinate system; S6, on the basis of the coordinates obtained in S5, calculating a second transformation matrix for the transformation between the electromagnetic space and the robot coordinate system; and S7, by means of the second transformation matrix, transforming the surgical operation information in the electromagnetic space into coordinates in the robot coordinate system, so as to perform navigation for the surgical tool.

## Description

For all purposes, this application claims the priority of Chinese Application No. 202311315344.7, filed on October 11, 2023, which is incorporated herein by reference in its entirety.

### TECHNICAL FIELD

The present disclosure relates to an electromagnetic navigation method for a surgical robot.

### BACKGROUND

Surgical robots are used to assist in surgical procedures, create customized three-dimensional preoperative plans, provide clearer and more precise visualization of the surgical site, and reduce tremors to improve surgical accuracy, minimize damage to healthy bones and tissues, reduce blood loss, protect nerves, shorten hospital stays and accelerate recovery. They also enable remote-guided surgeries and reduce the need for intraoperative imaging (e.g., X-rays) to lower radiation exposure.

Robot-assisted orthopedic surgery is a key application of orthopedic surgical robots. In the past, surgeons placed screws in the spine to perform complex spinal surgery either manually or with the assistance of numerous X-rays taken in surgery, exposing both patients and surgeons to radiation risks. Furthermore, a spinal surgery demands high precision in screw placement, and the consequence of placing screws in incorrect or suboptimal positions can be severe.

The orthopedic surgical robots provide a guidance system based on computerized preoperative planning, significantly improving accuracy and reducing the risk of screw malplacement. However, existing orthopedic surgical robots typically have only one or two arms used for intraoperative navigation and/or K-wire placement. In the conventional orthopedic robot assisted pedicle screw placement procedure, a guide wire is inserted according to the intraoperative planning, followed by the insertion of a hollow pedicle screw through the guide wire. However, the process ultimately relies on the surgeons to manually drill and insert the screw, resulting in low placement efficiency. Existing orthopedic surgical robots are generally equipped with an infrared navigation system, which is susceptible to obstruction by intraoperative objects and movement of patients or patient support apparatus, leading to navigation errors and surgical interruptions.

### SUMMARY

The technical problem to be solved by the present disclosure is to provide an electromagnetic navigation method for a surgical robot, whereby the positioning and navigation performance of the surgical robot is not affected by obstruction of an object within a surgical space.

The technical problem is solved by an electromagnetic navigation method for a surgical robot, and the electromagnetic navigation method includes the steps of:
S1: providing a reference frame apparatus including a reference frame body, a marking apparatus for displaying in an image captured by a medical imaging device, and a positioning sensor apparatus;
S2: performing imaging scanning on the reference frame apparatus by an intraoperative medical imaging device to obtain the coordinates of the marking apparatus in an intraoperative medical imaging device space;
S3: based on the coordinates of the marking apparatus in an electromagnetic space and the coordinates of the marking apparatus in the intraoperative medical imaging device space, calculating a first transformation matrix for the transformation from the coordinates in the electromagnetic space to the coordinates in the intraoperative medical imaging device space;
S4: transforming, by the first transformation matrix, surgical operation information in the intraoperative medical imaging device into surgical operation information in the electromagnetic space;
S5: acquiring the coordinates of a surgical instrument in the electromagnetic space and the coordinates of the surgical instrument in a robot coordinate system;
S6: based on the coordinates of the surgical instrument in both the electromagnetic space and the robot coordinate system, calculating a second transformation matrix for the transformation between the electromagnetic space and the robot coordinate system;
and S7: transforming, by the second transformation matrix, the surgical operation information in the electromagnetic space into the coordinates in the robot coordinate system, so as to perform navigation for the surgical instrument based on the coordinates. By the electromagnetic navigation method for an orthopedic surgical robot of the present disclosure, the positioning and navigation performance of the orthopedic surgical robot is not affected by obstruction of the object within the surgical space, thereby providing an orthopedic surgical robot with stable positioning and navigation performance.

An extended solution of the present disclosure provides that, the marking apparatus is designed as at least three tantalum markers arranged on the reference frame body, where the at least three tantalum markers are sequenced in the step S2, and in the step S3, the first transformation matrix is obtained, based on the sequenced at least three tantalum markers in the intraoperative medical imaging device space.

A further extended solution of the present disclosure provides that, based on the coordinates of the positioning sensor apparatus in the electromagnetic space and the coordinates of the marking apparatus in a positioning sensor apparatus space, the coordinates of the marking apparatus in the electromagnetic space are determined.

A further extended solution of the present disclosure provides that, the surgical operation information in the intraoperative medical imaging device is transformed from preoperative surgical operation information, and the preoperative surgical operation information is planned on a three-dimensional model by the preoperative medical imaging device for an orthopedic surgical operation.

A further extended solution of the present disclosure provides that, the reference frame body is designed as an implant to be implanted into a surgical site or as a clamping apparatus to be clamped onto a rigid tissue, and the positioning sensor apparatus is positioned closest to the surgical site. The positioning sensor apparatus of the present disclosure is disposed on the implant or clamping apparatus and accordingly is located within the surgical site, which differs from the situations in the prior art: in one such situation, a reference frame is positioned outside the surgical site; in another, a reference frame used for infrared navigation at present needs to be driven into the body for securing. Therefore, the electromagnetic navigation method according to the present disclosure can at least eliminate this cumbersome step, thereby simplifying the operational procedures. Furthermore, it can eliminate the need for a device designed solely as a reference frame, thereby saving device cost. According to the present disclosure, an electromagnetic positioning sensor apparatus disposed on the implant or clamping apparatus is positioned closest to the surgical site, thereby improving the positioning and navigation accuracy of the orthopedic surgical robot.

A further extended solution of the present disclosure provides that, the step S1 further includes a first calibration process for obtaining a first positional relationship matrix between the positioning sensor apparatus and the at least three tantalum markers.

A further extended solution of the present disclosure provides that, the first calibration process includes the steps of:
S11: providing a first calibration apparatus for holding the reference frame, on which a first calibration sensor apparatus is fixedly mounted;
S12: acquiring the coordinates of the positioning sensor apparatus and the first calibration sensor apparatus in the electromagnetic space, respectively;
S13: based on these coordinates, calculating a first calibration transformation matrix for the transformation from the first calibration sensor apparatus to the positioning sensor apparatus;
S14: based on the arrangement of the marking apparatus and the first calibration sensor apparatus in the first calibration apparatus, predetermining a positional relationship between the first calibration sensor apparatus and the marking apparatus;
and S15: based on the calculated first calibration transformation matrix and the predetermined positional relationship between the first calibration sensor apparatus and the marking apparatus, calculating a first positional relationship matrix between the positioning sensor apparatus and the at least three tantalum markers.

A further extended solution of the present disclosure provides that, the sequencing in the step S2 is implemented by
a: performing three-dimensional space binarization to extract regions of the at least three tantalum markers from an image captured by the intraoperative medical imaging device;
b: separating the regions of the at least three tantalum markers based on a binarization result to obtain the coordinates of the at least three tantalum markers in the intraoperative medical imaging device space;
c: obtaining the point coordinates for the respective tantalum markers and calculating the center coordinates among the at least three tantalum markers;
and d: based on a distance from each tantalum marker to the center coordinates, sequencing the at least three tantalum markers.

According to the present disclosure, there are two arrangements of an electromagnetic sensor apparatus for electromagnetic sensing of an orthopedic surgical instrument:
when a sensor apparatus for electromagnetic sensing of a surgical instrument is fixedly connected to the surgical instrument, fitting is performed by rotating with a tip of the surgical instrument as the origin, thereby acquiring the coordinates of the tip of the surgical instrument in a coordinate system of the sensor apparatus for electromagnetic sensing of the surgical instrument;
and when a sensor apparatus for electromagnetic sensing of a surgical instrument is fixedly connected to the surgical instrument robotic arm, it provides favorably that the sensor apparatus for electromagnetic sensing of the surgical instrument is fixedly connected to a portion of a surgical instrument robotic arm that is closest to the tip of the surgical instrument, while a distance between the sensor apparatus and the surgical instrument robotic arm is maintained at a value greater than 150 mm.

A further extended solution of the present disclosure provides that, the step S5 further includes a second calibration process for obtaining a transformation matrix between the coordinates of a distal end of a robotic arm of a robot and the coordinates of the sensor apparatus for electromagnetic sensing of the surgical instrument, where the second calibration process includes the steps of:
S21: providing a second calibration apparatus for calibrating the distal end of the surgical instrument robotic arm, on which a second calibration sensor apparatus is fixedly mounted, and detachably connecting the second calibration apparatus to a quick-release coupling of the surgical instrument robotic arm;
S22: acquiring the coordinates of the sensor apparatus for electromagnetic sensing of the surgical instrument and the second calibration sensor apparatus in the electromagnetic space, respectively;
S23: based on the acquired coordinates, calculating a second calibration transformation matrix for the transformation from the second calibration sensor apparatus to the sensor apparatus for electromagnetic sensing of the surgical instrument;
S24: based on the arrangement of the second calibration sensor apparatus in the second calibration apparatus, predetermining a positional relationship between the second calibration sensor apparatus and the distal end of the robotic arm;
and S25: based on the calculated second calibration transformation matrix and the predetermined positional relationship between the second calibration sensor apparatus and the distal end of the robotic arm, calculating a second positional relationship matrix between the sensor apparatus for electromagnetic sensing of the surgical instrument and the distal end of the surgical instrument robotic arm.

A further extended solution of the present disclosure provides that, based on the distal end of the surgical instrument robotic arm and the surgical instrument mounted on the distal end of the surgical instrument robotic arm, a positional relationship between the distal end of the surgical instrument robotic arm and the tip of the surgical instrument is obtained, where structural dimensions of the surgical instrument are predetermined.

A further extended solution of the present disclosure provides that, based on the positional relationship between the distal end of the surgical instrument robotic arm and the tip of the surgical instrument, as well as the second positional relationship matrix, the coordinates of the tip of the surgical instrument in the coordinate system of the sensor apparatus for electromagnetic sensing of the surgical instrument are obtained.

The following detailed description of the examples, in conjunction with the accompanying drawings, will clarify further details and advantages of the examples described herein.

### BRIEF DESCRIPTION OF DRAWINGS

Fig. 1 is a flowchart illustrating an electromagnetic navigation method for an orthopedic robot according to a non-restrictive embodiment of the present disclosure;
Fig. 2 is a flowchart illustrating a first calibration process according to a non-restrictive embodiment of the present disclosure;
Fig. 3 is a flowchart illustrating the sequencing of tantalum rods in an image captured by an intraoperative medical imaging device;
Fig. 4 is a flowchart illustrating a second calibration process according to a non-restrictive embodiment of the present disclosure.

### DETAILED DESCRIPTION

A medical imaging device of the present disclosure may include a computed tomography (CT) scanner, an X-ray imaging device, a magnetic resonance imaging (MRI) device and a nuclear medicine device. The following description of the electromagnetic navigation method for an orthopedic surgical robot is based merely on a preferred CT device as an example. A tantalum marker may be designed into various forms, such as a tantalum rod, a tantalum sphere, and a tantalum wire. The present disclosure describes the electromagnetic navigation method for an orthopedic surgical robot by using the tantalum rod as a preferred example. The term "center coordinates" represents the x, y, and z coordinates of the center of multiple points in a three-dimensional space, such as the center of four points. For this center, x-coordinate = (x1 + x2 + x3 + x4) / 4, y-coordinate = (y1 + y2 + y3 + y4) / 4, and z-coordinate = (z1 + z2 + z3 + z4) / 4, where xn represents the x-coordinate of a nth point, yn represents the y-coordinate of the nth point, and zn represents the z-coordinate of the nth point, with n = 1, 2, 3, 4.

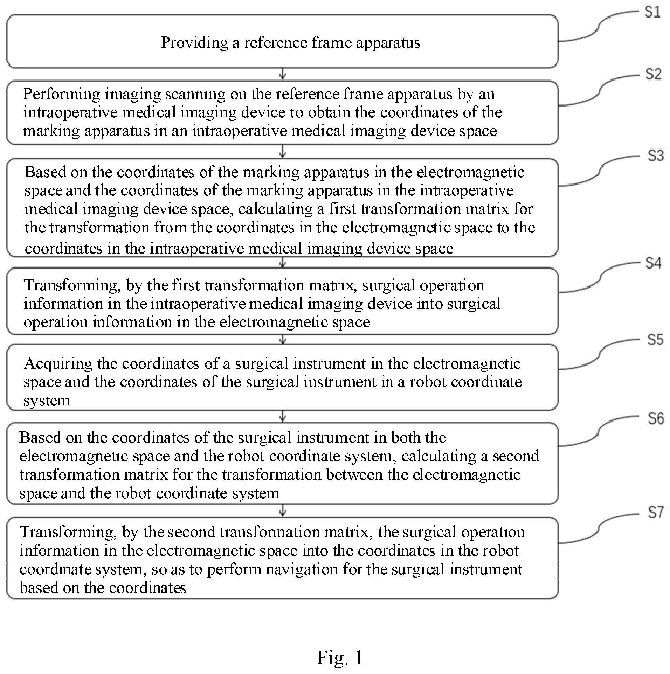

Fig. 1 is a flowchart illustrating an electromagnetic navigation method for an orthopedic robot according to a non-restrictive embodiment of the present disclosure. As shown in Fig. 1, the electromagnetic navigation method for an orthopedic robot according to the present disclosure includes the following steps.

Step S1: providing a reference frame apparatus which includes a reference frame body, a positioning sensor apparatus, and a marking apparatus for displaying in an image captured by a medical imaging device, especially by a CT device, where the reference frame body is designed as an implant to be implanted into a surgical site or as a clamping apparatus to be clamped onto a rigid tissue, and the positioning sensor apparatus is positioned closest to the surgical site, so that errors caused by patient movement and other factors can be reduced, significantly improving the positioning and navigation accuracy of the orthopedic surgical robot. The implant may be designed as an interbody fusion device for providing interbody support and promoting fusion during a fusion surgery, or may be designed as a K-wire. The clamping apparatus may be designed as a spinous process clamp to be clamped onto the rigid tissue closest to the surgical site. In this way, the electromagnetic navigation method of the present disclosure can eliminate at least the cumbersome step in the prior art where a reference frame needs to be inserted into the body for securing, thereby simplifying the surgical procedures. Moreover, it can eliminate the need for a device designed solely as a reference frame, thereby saving device cost. The marking apparatus on the reference frame apparatus may be designed as at least three tantalum markers, specifically the coordinates of tantalum rods in an electromagnetic space, which are arranged on the reference frame body. The positioning sensor apparatus on the reference frame apparatus may be designed as one that is mounted on the reference frame body by a detachable connection, where the detachable connection is preferably a threaded connection or a snap-fit connection.

In the step of providing the reference frame apparatus, the positioning sensor apparatus is first screwed onto the reference frame body via threads. However, because the position where the threads are screwed into the reference frame body changes each time, that is, the position where the positioning sensor apparatus is screwed into the corresponding threaded hole on the reference frame body changes each time, a positional relationship between each tantalum rod and the positioning sensor apparatus cannot be obtained only based on a structural positional relationship between the components. In view of this, a first calibration apparatus is needed for obtaining the positional relationship. Therefore, after the reference frame apparatus is prepared, it is further placed into the first calibration apparatus that is used for obtaining a first positional relationship matrix between the tantalum rod and the positioning sensor apparatus. The first calibration apparatus is provided with a first calibration sensor apparatus for obtaining registration information, in order to perform a first calibration process which will be described in detail below, where the registration information includes, for example, the coordinates of at least three and especially four tantalum rods in a positioning sensor apparatus space, i.e., positions of the tantalum rods relative to the positioning sensor apparatus; and moreover, the coordinates of the tantalum rod in the electromagnetic space can be obtained based on the coordinates of the tantalum rod in the positioning sensor apparatus space and the coordinates of the positioning sensor apparatus in the electromagnetic space.

Step S2: performing imaging scanning on the reference frame apparatus by an intraoperative medical imaging device to obtain the coordinates of the marking apparatus in an intraoperative medical imaging device space, where the intraoperative medical imaging device is preferably designed as a three-dimensional CT device, and in this embodiment, the marking apparatus is designed as at least three tantalum rods; accordingly, in order to distinguish between these at least three tantalum rods during coordinate transformation, the tantalum rods need to be sequenced to achieve the transformation in one to one correspondence between the coordinates of each rod in a CT space and the coordinates in the electromagnetic space.

Step S3: based on the coordinates of the marking apparatus, especially the tantalum rods, in the electromagnetic space and the coordinates of the marking apparatus in the intraoperative medical imaging device space, calculating a first transformation matrix for the transformation from the coordinates in the electromagnetic space to the coordinates in the intraoperative medical imaging device space, where the coordinates of each tantalum rod in the electromagnetic space are calculated based on the coordinates of the tantalum rod relative to the positioning sensor apparatus and the coordinates of the positioning sensor apparatus in the electromagnetic space.

Step S4: transforming, by the first transformation matrix, surgical operation information in the intraoperative medical imaging device into surgical operation information in the electromagnetic space, where the surgical operation information may include, for example, the drill position, screw insertion point, and drill and insertion depth; the surgical operation information in the intraoperative medical imaging device may optionally be transformed from preoperative surgical operation information or obtained directly from intraoperative surgical operation information; the preoperative surgical operation information refers to information planned on a three-dimensional model by the preoperative medical imaging device for an orthopedic surgical operation, and the intraoperative surgical operation information refers to information planned on a three-dimensional model by a three-dimensional medical imaging device for an orthopedic surgical operation.

According to an non-restrictive embodiment of the present disclosure, in the step S4, the preoperative planned information on the three-dimensional model for an orthopedic surgical operation, such as the drill position, screw insertion point and screw depth, is obtained from a preoperative CT to perform preoperative and intraoperative CT point cloud registration, in order to transform the information in a preoperative CT image for an orthopedic surgical operation into an intraoperative CT image. Here, the preoperative CT image is used to record the preoperative planned element such as the drill position, screw insertion point and screw depth in the surgical site; the intraoperative CT image is used to record the drill position, screw insertion point and screw depth in an intraoperative pose in the surgical site. By registering the intraoperative CT with the preoperative CT, the coordinates of the preoperative planned element such as the drill position and screw insertion point in a preoperative CT space are transformed into the coordinates of the preoperative planned element such as the drill position and screw insertion point in an intraoperative CT space; and then, by the obtained transformation matrix for the electromagnetic and CT coordinates, the coordinates of the intraoperative drill position, screw insertion point, etc., in the intraoperative CT space are transformed into the coordinates of the drill position, screw insertion point, etc., in the electromagnetic space.

Step S5: acquiring the coordinates of a surgical instrument in the electromagnetic space and the coordinates of the surgical instrument in a robot coordinate system, where the coordinates of the surgical instrument in the robot coordinate system is obtained by taking a component of the orthopedic robot, such as a robotic arm base, as the origin of the robot coordinate system; the coordinates of the surgical instrument in the electromagnetic space, especially the coordinates of a tip of an osteotome or screw mounting instrument in the electromagnetic space, are used for navigation of the osteotome or screw mounting instrument based on the obtained information such as the coordinates of the drill position in the electromagnetic space; the method for obtaining the coordinates of the surgical instrument in the electromagnetic space, as described in the present disclosure, depends on the type of the sensor apparatus for electromagnetic sensing of the surgical instrument. According to a non-restrictive embodiment of the present disclosure, there are two arrangements of a sensor apparatus for electromagnetic sensing of an orthopedic surgical instrument.

A first arrangement involves fixedly connecting an electromagnetic sensor apparatus for electromagnetic sensing of the orthopedic surgical instrument, such as an electromagnetic position tracker, to the surgical instrument, such as the osteotome. By manually rotating the osteotome, coordinate information of a tip of the osteotome within a coordinate system of the sensor apparatus for electromagnetic sensing of the surgical instrument is generated, where "rotating" refers to rotating the osteotome while holding it with its tip as the origin, and then fitting is performed to obtain tip pose information of the osteotome, i.e., the coordinates of the osteotome, especially its tip, within the coordinate system of the sensor apparatus for electromagnetic sensing of the surgical instrument, thereby enabling positioning and navigation of the osteotome in the electromagnetic space.

A second arrangement involves fixedly connecting an sensor apparatus for electromagnetic sensing of the orthopedic surgical instrument to this position on a surgical instrument robotic arm, where at this position, the sensor apparatus for electromagnetic sensing of the surgical instrument is located closest to the tip of the surgical instrument and has a distance from the surgical instrument robotic arm greater than 150 mm, thereby avoiding electromagnetic interference caused by a motor of the surgical instrument robotic arm. To perform registration or transformation between the coordinates of a distal end of the surgical instrument robotic arm and the coordinates of the sensor apparatus for electromagnetic sensing of the surgical instrument, the present disclosure provides for a second calibration process using a second calibration apparatus after the sensor apparatus for electromagnetic sensing of the surgical instrument has been installed, where the second calibration apparatus is designed to include a second calibration sensor apparatus, such as a disc-shaped sensor apparatus, which is fixedly positioned on the second calibration apparatus; and moreover, the second calibration apparatus is designed to be detachably connected to a quick-release coupling of the surgical instrument robotic arm for performing the second calibration process, as will be described in detail below.

Step S6: based on the coordinates of the surgical instrument in both the electromagnetic space and the robot coordinate system, calculating a second transformation matrix for the transformation between the electromagnetic space and the robot coordinate system, where the coordinates of the surgical instrument in the robot coordinate system, specifically the coordinates of the tip of the osteotome/screw in the robot coordinate system, can still be obtained by using the robotic arm base as the origin of the robot coordinate system, in conjunction with the structural dimensions of the osteotome or screw.

Step S7: transforming, by the second transformation matrix, the surgical operation information in the electromagnetic space into the coordinates in the robot coordinate system, so as to perform navigation for the surgical instrument based on the coordinates, where a second registration matrix for the registration between an electromagnetic space coordinate system and the robot coordinate system is calculated based on the coordinates of the surgical instrument in both the electromagnetic space and the robot coordinate system, that is, the registration is performed according to results in S5 and S6 to obtain the second transformation matrix for the transformation from the electromagnetic space coordinate system to the robot coordinate system.

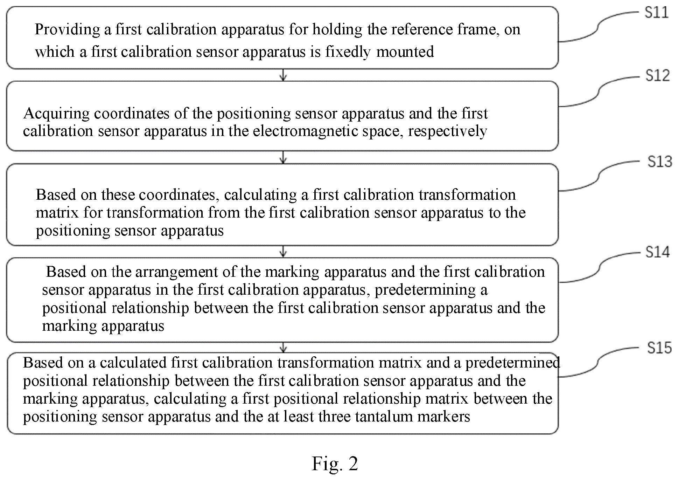

Fig. 2 is a flowchart illustrating a first calibration process according to a non-restrictive embodiment of the present disclosure. A first calibration positional relationship matrix between the positioning sensor apparatus and, for example, four tantalum rods can be obtained through the first calibration process. The first calibration process includes the following steps.
S11: providing a calibration apparatus for calibrating the reference frame, especially for an interbody fusion apparatus, where the calibration apparatus is provided with a positioning apparatus for positioning the reference frame and is fixedly equipped with a calibration sensor apparatus; additionally, after the reference frame is positioned on the calibration apparatus, the following step may be performed: pressing the reference frame downward to abut against the calibration apparatus by an additional pressing apparatus and holding the reference frame in place to prevent the reference frame from moving in a height direction of the positioning device as the calibration apparatus moves;
S12: under an electromagnetic field for electromagnetic positioning and navigation, acquiring the coordinates of the positioning sensor apparatus and the first calibration sensor apparatus in the electromagnetic space, respectively;
S13: based on the acquired coordinates, calculating a first calibration transformation matrix for the transformation from the first calibration sensor apparatus to the positioning sensor apparatus;
S14: based on the predetermined arrangement of the four tantalum rods and the first calibration sensor apparatus in the first calibration apparatus, determining a positional relationship between the first calibration sensor apparatus and the four tantalum rods;
and S15: based on the calculated first calibration transformation matrix and the predetermined positional relationship between the first calibration sensor apparatus and the four tantalum rods, calculating a positional relationship matrix between the positioning sensor apparatus and the four tantalum rods. To obtain a more accurate first calibration positional relationship matrix, after the step 15, step S16 is performed: moving the calibration apparatus in the electromagnetic field for electromagnetic positioning and navigation to obtain multiple different poses of the calibration apparatus, where the steps S12-S15 are repeated for each pose to obtain multiple positional relationship matrices between the positioning sensor apparatus and the marking apparatus, and then solving an average positional relationship matrix from the multiple positional relationship matrices.

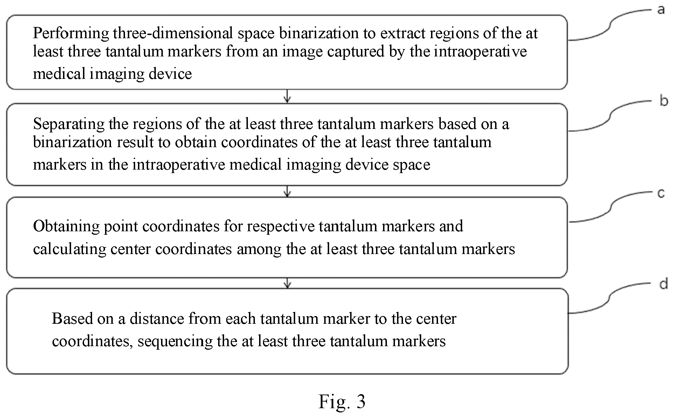

Fig. 3 is a flowchart illustrating the sequencing of the marking apparatus, especially the tantalum rods, in an image captured by the intraoperative medical imaging device. The sequencing includes the following steps:
a: performing three-dimensional space binarization to extract a tantalum rod region from an intraoperative CT image, where the CT image obtained by scanning of a CT device contains multiple subregions, these subregions are converted into images with a value of only 1 or 0 based on their grayscale values; in response to the actual grayscale value of a subregion exceeding a grayscale threshold used to identify the tantalum rod region, the value of that region is set to 1, e.g., representing a tantalum rod; in response to the actual grayscale value of a subregion being less than the grayscale threshold used to identify the tantalum rod region, the value of that region is set to 0, e.g., representing a background;
b: separating the tantalum rod region based on a binarization result to obtain the coordinates of the tantalum rod in the CT space;
c: obtaining at least three sets, especially four sets, of point coordinates, i.e., the coordinates of at least three, especially four, tantalum rods (center points of the tantalum rods themselves) in the CT space, and calculating the center coordinates among the at least three, especially four, tantalum rods;
and d: performing sequencing of the at least three, especially four, tantalum rods to distinguish them, where the sequencing criterion may, for example, be a distance between the point coordinates corresponding to the center point of each tantalum rod itself and the center coordinates. Of course, the tantalum rods may also be sequenced according to other criteria, provided they do not conflict with the solutions of the present disclosure.

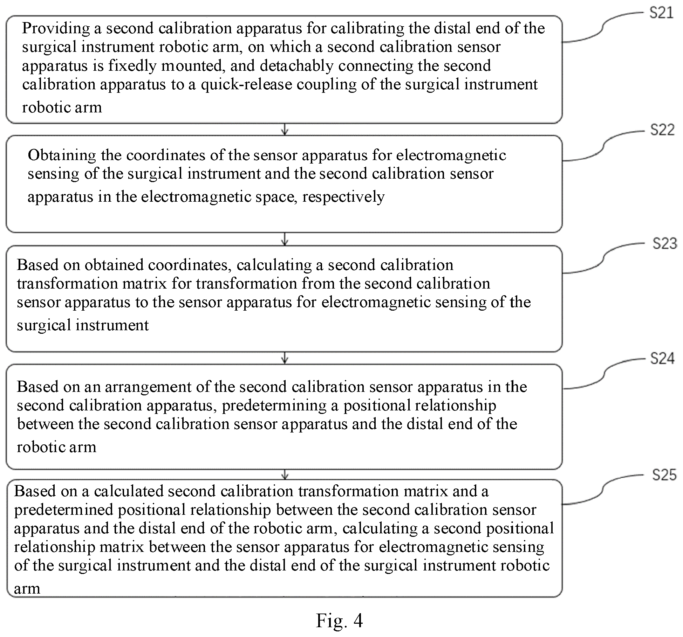

Fig. 4 is a flowchart illustrating a second calibration process according to a non-restrictive embodiment of the present disclosure. When the sensor apparatus for electromagnetic sensing of the surgical instrument is disposed on the surgical instrument robotic arm, the electromagnetic navigation method for an orthopedic surgical robot further includes a second calibration process for obtaining a transformation matrix between the coordinates of a distal end of a robotic arm of a robot and the coordinates of the sensor apparatus for electromagnetic sensing of the surgical instrument. The second calibration process includes the following steps:
S21: providing a second calibration apparatus for calibrating the distal end of the surgical instrument robotic arm, on which a second calibration sensor apparatus is fixedly mounted, and detachably connecting the second calibration apparatus to a quick-release coupling of the surgical instrument robotic arm;
S22: acquiring the coordinates of the sensor apparatus for electromagnetic sensing of the surgical instrument and the second calibration sensor apparatus in the electromagnetic space, respectively;
S23: based on the acquired coordinates, calculating a second calibration transformation matrix for the transformation from the second calibration sensor apparatus to the sensor apparatus for electromagnetic sensing of the surgical instrument;
S24: based on the arrangement of the second calibration sensor apparatus in the second calibration apparatus, predetermining a positional relationship between the second calibration sensor apparatus and the distal end of the robotic arm;
and S25: based on the calculated second calibration transformation matrix and the predetermined positional relationship between the second calibration sensor apparatus and the distal end of the robotic arm, calculating a second positional relationship matrix between the sensor apparatus for electromagnetic sensing of the surgical instrument and the distal end of the surgical instrument robotic arm.

The aforementioned electromagnetic navigation method for a surgical robot is particularly effective in ensuring that the positioning and navigation of the orthopedic surgical robot are not affected by obstruction of an object within the surgical space.

Although examples of the present disclosure have been provided in the preceding description, those skilled in the art may make modifications and variations of these examples without departing from the scope and spirit of the present disclosure. For example, it should be understood that features of the embodiments described herein may be applied to other embodiments described herein. Therefore, the description is intended to be illustrative rather than limiting. The disclosure is defined by the appended claims, and all changes to the disclosure falling within the meaning and scope of the claims are included within their scope.

## Claims

1. An electromagnetic navigation method for a surgical robot, comprising the steps of:
S1: providing a reference frame apparatus comprising a reference frame body, a marking apparatus for displaying in an image captured by a medical imaging device, and a positioning sensor apparatus;
S2: performing imaging scanning on the reference frame apparatus by an intraoperative medical imaging device to obtain coordinates of the marking apparatus in an intraoperative medical imaging device space;
S3: based on coordinates of the marking apparatus in an electromagnetic space and the coordinates of the marking apparatus in the intraoperative medical imaging device space, calculating a first transformation matrix for transformation from the coordinates in the electromagnetic space to the coordinates in the intraoperative medical imaging device space;
S4: transforming, by the first transformation matrix, surgical operation information in the intraoperative medical imaging device into surgical operation information in the electromagnetic space;
S5: acquiring coordinates of a surgical instrument in the electromagnetic space and coordinates of the surgical instrument in a robot coordinate system;
S6: based on the coordinates of the surgical instrument in both the electromagnetic space and the robot coordinate system, calculating a second transformation matrix for transformation between the electromagnetic space and the robot coordinate system; and
S7: transforming, by the second transformation matrix, the surgical operation information in the electromagnetic space into the coordinates in the robot coordinate system, so as to perform navigation for the surgical instrument based on the coordinates.

2. The method according to claim 1, wherein the marking apparatus is designed as at least three tantalum markers arranged on the reference frame body, the at least three tantalum markers are sequenced in the step S2, and in the step S3, the first transformation matrix is obtained, based on sequenced at least three tantalum markers in the intraoperative medical imaging device space.

3. The method according to claim 1 or 2, wherein based on coordinates of the positioning sensor apparatus in the electromagnetic space and coordinates of the marking apparatus in a positioning sensor apparatus space, coordinates of the marking apparatus in the electromagnetic space are determined.

4. The method according to claim 1 or 2, wherein the reference frame body is designed as an implant to be implanted into a surgical site or as a clamping apparatus to be clamped onto a rigid tissue, and the positioning sensor apparatus is positioned closest to the surgical site.

5. The method according to claim 2, wherein the step S1 further comprises a first calibration process for obtaining a first positional relationship matrix between the positioning sensor apparatus and the at least three tantalum markers.

6. The method according to claim 5, wherein the first calibration process comprises the steps of:
S11: providing a first calibration apparatus for holding the reference frame, on which a first calibration sensor apparatus is fixedly mounted;
S12: acquiring coordinates of the positioning sensor apparatus and the first calibration sensor apparatus in the electromagnetic space, respectively;
S13: based on these coordinates, calculating a first calibration transformation matrix for transformation from the first calibration sensor apparatus to the positioning sensor apparatus;
S14: based on the arrangement of the marking apparatus and the first calibration sensor apparatus in the first calibration apparatus, predetermining a positional relationship between the first calibration sensor apparatus and the marking apparatus; and
S15: based on a calculated first calibration transformation matrix and a predetermined positional relationship between the first calibration sensor apparatus and the marking apparatus, calculating a first positional relationship matrix between the positioning sensor apparatus and the at least three tantalum markers.

7. The method according to claim 2, wherein the sequencing in the step S2 is implemented by
a: performing three-dimensional space binarization to extract regions of the at least three tantalum markers from an image captured by the intraoperative medical imaging device;
b: separating the regions of the at least three tantalum markers based on a binarization result to obtain coordinates of the at least three tantalum markers in the intraoperative medical imaging device space;
c: obtaining point coordinates for respective tantalum markers and calculating center coordinates among the at least three tantalum markers; and
d: based on a distance from each tantalum marker to the center coordinates, sequencing the at least three tantalum markers.

8. The method according to claim 1 or 2, wherein when a sensor apparatus for electromagnetic sensing of a surgical instrument is fixedly connected to the surgical instrument, fitting is performed by rotating with a tip of the surgical instrument as an origin, thereby acquiring coordinates of the tip of the surgical instrument in a coordinate system of the sensor apparatus for electromagnetic sensing of the surgical instrument.

9. The method according to claim 1 or 2, wherein a sensor apparatus for electromagnetic sensing of a surgical instrument is fixedly connected to a portion of a surgical instrument robotic arm that is closest to a tip of the surgical instrument, while a distance between the sensor apparatus and the surgical instrument robotic arm is maintained at a value greater than 150 mm.

10. The method according to claim 9, wherein the step S5 further comprises a second calibration process for obtaining a transformation matrix between coordinates of a distal end of a robotic arm of a robot and coordinates of the sensor apparatus for electromagnetic sensing of the surgical instrument, wherein the second calibration process comprises the steps of:
S21: providing a second calibration apparatus for calibrating the distal end of the surgical instrument robotic arm, on which a second calibration sensor apparatus is fixedly mounted, and detachably connecting the second calibration apparatus to a quick-release coupling of the surgical instrument robotic arm;
S22: obtaining the coordinates of the sensor apparatus for electromagnetic sensing of the surgical instrument and the second calibration sensor apparatus in the electromagnetic space, respectively;
S23: based on obtained coordinates, calculating a second calibration transformation matrix for transformation from the second calibration sensor apparatus to the sensor apparatus for electromagnetic sensing of the surgical instrument;
S24: based on an arrangement of the second calibration sensor apparatus in the second calibration apparatus, predetermining a positional relationship between the second calibration sensor apparatus and the distal end of the robotic arm; and
S25: based on a calculated second calibration transformation matrix and a predetermined positional relationship between the second calibration sensor apparatus and the distal end of the robotic arm, calculating a second positional relationship matrix between the sensor apparatus for electromagnetic sensing of the surgical instrument and the distal end of the surgical instrument robotic arm.

11. The method according to claim 9, wherein based on the distal end of the surgical instrument robotic arm and the surgical instrument mounted on the distal end of the surgical instrument robotic arm, a positional relationship between the distal end of the surgical instrument robotic arm and the tip of the surgical instrument is obtained.

12. The method according to claim 11, wherein based on the positional relationship between the distal end of the surgical instrument robotic arm and the tip of the surgical instrument, as well as the second positional relationship matrix, coordinates of the tip of the surgical instrument in a coordinate system of the sensor apparatus for electromagnetic sensing of the surgical instrument are obtained.
